# EUROPÄISCHE PATENTSCHRIFT

(11) **EP 0 633 246 B1**
(45) Veröffentlichungstag und Bekanntmachung des Hinweises auf die Patenterteilung: **14.03.2001**
(21) Anmeldenummer: 94109956.6
(22) Anmeldetag: 28.06.1994
(51) Int. Cl.: C07D 201/16, C07D 201/12

(54) **Verfahren zur Gewinnung von Caprolactam und eines Alkalimetallcarbonats aus Destillationsrückständen, die bei der Reinigung von Caprolactam anfallen**
Process for the recovery of caprolactam and an alkaline metal carbonate from distillation residues formed during the purification of caprolactam
Procédé d'obtention de caprolactame et d'un carbonate de métal alcalin à partir de résidus de distillation formés au cours de la purification de caprolactame

(30) Priorität: 09.07.1993 DE 4322953
(43) Veröffentlichungstag der Anmeldung: 11.01.1995
(73) Patentinhaber: BASF Aktiengesellschaft, 67063 Ludwigshafen (DE)
(72) Erfinder: Fuchs, Hugo, Dr., D-67071 Ludwigshafen (DE); Ritz, Josef, Dr., D-67069 Ludwigshafen (DE); Wilfinger, Hans Joerg, Dr., D-67105 Schifferstadt (DE); Weatherford, David, Dr., D-68199 Mannheim (DE)

(56) Entgegenhaltungen:
- EP-A- 0 306 874
- DE-C- 950 726
- US-A- 4 582 642
- CHEMICAL ABSTRACTS, vol. 76, no. 6, 7. Februar 1972, Columbus, Ohio, US; abstract no. 25764j, & JP-A-46 024 388 (TOYO SPINNING CO., LTD.) 13. Juli 1971
- DATABASE WPI Week 7743, Derwent Publications Ltd., London, GB; AN 77-76353Y & JP-A-50 123 690 (KANEBO K.K.) 29. September 1975

## Beschreibung

Die vorliegende Erfindung betrifft ein Verfahren zur Gewinnung von Caprolactam und eines Alkalimetallcarbonats aus Destillationsrückständen, die bei der Reinigung von Caprolactam anfallen und Caprolactam und Oligomere und Polymere des Caprolactams sowie Alkalimetallhydroxide enthalten.

Caprolactam wird im allgemeinen bei dessen Herstellung zum Zwecke der Reinigung durch Destillation von leicht- und schwersiedenden Produkten getrennt.

Die hierbei anfallenden schwersiedenden Destillationsrückstände enthalten in der Regel noch Caprolactam, dessen Oligomere und Polymere sowie weitere Verunreinigungen und Zersetzungsprodukte. Bei der Destillation in Gegenwart von Alkalimetall-haltigen Verbindungen, sind auch diese in den Rückständen üblicherweise meist in Form ihrer Hydroxide enthalten.

Solche Destillationsrückstände können einen relativ hohen Anteil an Oligomeren des Caprolactams enthalten, wenn das Caprolactam, das aus einer Spaltanlage erhalten wird, gemeinsam mit frisch hergestelltem Caprolactam aufgearbeitet wird.

In der EP-A 306 874 wird ein Verfahren zur Aufarbeitung von Destillationsrückständen, die bei der Reinigung von Caprolactam anfallen, beschrieben, wobei die Destillationsrückstände in Gegenwart von Natrium- und Kaliumhydroxid und in Gegenwart eines hochsiedenden Kohlenwasserstoffs auf eine Temperatur von 250 bis 500°C erhitzt und Caprolactam fortlaufend aus dem Reaktionsgemisch entfernt werden. Nachteilig an diesem Verfahren ist, daß der hierbei anfallende Rückstand nur schwer weiterverarbeitbar ist und im allgemeinen zur Entfernung aus dem Reaktionsgefäß in handhabbare Stücke geschlagen werden muß. Ferner erhält man üblicherweise bei dem Versuch diesen Rückstand in Wasser zu lösen eine zähflüssige Suspension. Sowohl eine Entsorgung dieser Suspension über eine biologische Kläranlage als auch eine Deponierung sind bislang unwirtschaftlich. Ein weiterer Nachteil bei dem Verfahren der EP-A 306 874 ist, daß die verwendeten hochsiedenden Kohlenwasserstoffe im allgemeinen gereinigt und in einigen Fällen sogar durch Verbrennung entsorgt werden müssen.

Aufgabe der vorliegenden Erfindung war es daher, ein Verfahren zur Gewinnung von Caprolactam und eines Alkalimetallcarbonats aus Destillationsrückständen, die bei der Reinigung von Caprolactam anfallen und Caprolactam und Oligomere und Polymere des Caprolactams sowie Alkalimetallhydroxide enthalten, zur Verfügung zu stellen, bei dem der Einsatz hochsiedender Kohlenwasserstoffe vermieden und der anfallende Rückstand in eine wasserlösliche Form gebracht wird unter Gewinnung eines wiederverwertbaren Alkalimetallcarbonats.

Demgemäß wurde ein Verfahren zur Gewinnung von Caprolactam und eines Alkalimetallcarbonats aus Destillationsrückständen, die bei der Reinigung von Caprolactam anfallen und Caprolactam und Oligomere und Polymere des Caprolactams sowie Alkalimetallhydroxide enthalten, gefunden, indem man
(a) die Destillationsrückstände zur Schmelze Schmelze bringt,
(b) Caprolactam fortlaufend aus der Schmelze mit überhitztem Wasserdampf und/oder Inertgas entfernt,
(c) nach dem Entfernen von Caprolactam die Schmelze abkühlt unter Erhalt eines Rückstandes,
(d) den so erhaltenen Rückstand in Wasser löst und
(e) die so erhaltene wäßrige Lösung der Verbrennung unter Bildung eines Alkalimetallcarbonats unterwirft.

Erfindungsgemäß geht man von Destillationsrückständen aus, die bei der Reinigung von Caprolactam anfallen und Caprolactam und Oligomere und Polymere des Caprolactams sowie mindestens ein A1-kalimetallhydroxid enthalten. Solche Destillationsrückstände erhält man beispielsweise nach destillativem Abtrennen von Caprolactam in Gegenwart eines Alkalimetallhydroxids bei Sumpftemperaturen im Bereich von 120 bis 150°C unter vermindertem Druck, beispielsweise bei einem Druck im Bereich von 1 bis 50 mbar.

Ein erfindungsgemäß geeigneter Destillationsrückstand hat in der Regel die folgende Zusammensetzung:
Caprolactam: von 1 bis 60, bevorzugt von 10 bis 50 Gew.-%
Oligomere und Polymere des Caprolactams: von 1 bis 30, bevorzugt von 5 bis 20 Gew.-%
Alkalimetallhydroxid: von 1 bis 20, bevorzugt von 1 bis 10 Gew.-%
Rest (Verunreinigungen und Zersetzungsprodukte): 100 - Summe der oben genannten Bestandteile
Den Destillationsrückstand bringt man in der Regel zur Schmelze, indem man ihn auf Temperaturen im Bereich von 120 bis 400, vorzugsweise von 150 bis 350°C bei Drücken im Bereich von 20 bis 1013 hPa erhitzt.

Erfindungsgemäß entfernt man Caprolactam fortlaufend aus der Schmelze. Um dies zu erreichen, leitet man überhitzten Wasserdampf und/oder Inertgase wie Stickstoff, Kohlendioxid, Edelgase oder Rauchgase durch die Schmelze. Besonders geeignet sind überhitzter Wasserdampf und Stickstoff. Je kg Destillationsrückstand leitet man üblicherweise 10 bis 6000 l/h, vorzugsweise 1000 bis 3000 l/h Gas durch die Schmelze. Insbesondere verwendet man bei Verwendung von überhitztem Wasserdampf je kg Schmelze 0,1 bis 20 Gewichtsteile Wasserdampf.

In einer bevorzugten Ausführungsform entfernt man Caprolactam fortlaufend durch Anlegen eines Unterdrucks, d.h. einem Druck im Bereich von 20 bis 1013, vorzugsweise von 20 bis 100 hPa, durch zusätzliche Mitverwendung von überhitztem Wasserdampf und/oder Inertgasen aus der Schmelze.

Das aus der Schmelze gewonnene Caprolactam wird in der Regel durch Kühlen kondensiert oder durch Waschen mit einem geeigneten Lösungsmittel wie Wasser abgeschieden. Überhitzter Wasserdampf und/oder Inertgase können zweckmäßig zurückgeführt werden.

Das erfindungsgemäß erhaltene Caprolactam kann man gewünschtenfalls durch übliche Maßnahmen wie Destillation reinigen und/oder sogenanntem Rohlactam (d.h. keinem reinen Caprolactam), das bei der Beckmannschen Umlagerung nach dessen Neutralisation anfällt, zuführen.

Nach dem Entfernen des Caprolactams laßt man erfindungsgemäß die Schmelze abkühlen und löst den abgekühlten Rückstand bei einer Temperatur, die üblicherweise im Bereich von 20 bis 100, vorzugsweise von 30 bis 80°C liegt, in Wasser. Das Gewichtsverhältnis von Rückstand zu Wasser liegt im allgemeinen im Bereich von 0,05:1 bis 10:1, vorzugsweise von 0,1:1 bis 2:1.

Die erfindungsgemäß erhaltene wäßrige Lösung wird bei Temperaturen, die in der Regel im Bereich von 900 bis 1200, bevorzugt von 1000 bis 1100°C liegen, unter Erhalt eines Alkalimetallcarbonats wie Soda oder Pottasche oder einer Mischung davon in einer an sich bekannten Verbrennungsanlage verbrannt.

Die Reinheit der erfindungsgemäß erhaltenen Alkalimetallcarbonate wie Soda- oder Pottasche beträgt im allgemeinen mindestens 99,5 %.

Das erfindungsgemäß erhaltene Alkalimetallcarbonat ist ein vielseitig verwendbarer Ausgangsstoff beispielsweise für die Glasindustrie und die Seifenindustrie. Insbesondere kann Soda als Ersatz für Natriumhydroxid dienen.

Die Vorteile der vorliegenden Erfindung bestehen darin, daß gegenüber der EP-A 306 874 als nächstliegendem Stand der Technik neben der Gewinnung von Caprolactam der Einsatz hochsiedender Kohlenwasserstoffe vermieden und der anfallende Rückstand in eine wasserlösliche Form gebracht wird unter Gewinnung eines wiederverwertbaren Alkalimetallcarbonats.

### Beispiele

### Beispiel 1

195 g eines Destillationsrückstandes aus der Caprolactamreinigung, enthaltend 46,3 Gew.-% Caprolactam, 5 Gew.-% Natriumhydroxid sowie Oligomere, Verunreinigungen und Zersetzungsprodukte, wurden auf 315°C erhitzt und so zur Schmelze gebracht. Dann wurden 600 g 350°C heißer Wasserdampf im Laufe von 5 h durch die Schmelze geleitet unter Erhalt von 62 g eines wasserlöslichen Rückstandes. Der Rückstand wurde in 150 ml Wasser bei Raumtemperatur aufgenommen und anschließend durch Einsprühen in eine 1000°C heiße Flamme verbrannt. Als Verbrennungsrückstand wurden 12,5 g Soda erhalten. Das aus dem Reaktionsgefäß entweichende Wasserdampf/Caprolactam-Gemisch wurde kondensiert unter Erhalt von 133 g Caprolactam.

### Beispiel 2

521 g eines Destillationsrückstandes aus der Caprolactamreinigung, enthaltend 46,3 Gew.-% Caprolactam, 5,3 Gew.-% Natriumhydroxid sowie Oligomere, Verunreinigungen und Zersetzungsprodukte, wurden auf 340°C erhitzt und so zur Schmelze gebracht. Dann wurden innerhalb von 2 h 240 ml 340°C heißer Wasserdampf (Durchflußgeschwindigkeit: 0,12 l/h) durch die Schmelze geleitet unter Erhalt eines wasserlöslichen Rückstandes. Der Rückstand wurde in 350 ml Wasser bei 50°C aufgenommen und anschließend durch Einsprühen in eine 1000°C heiße Flamme verbrannt. Als Verbrennungsrückstand wurden 35 g Soda erhalten. Das aus dem Reaktionsgefäß entweichende Wasserdampf/Caprolactam-Gemisch wurde kondensiert unter Erhalt von 396 g Caprolactam.

## Patentansprüche

1. Verfahren zur Gewinnung von Caprolactam und eines Alkalimetallcarbonats aus Destillationsrückständen, die bei der Reinigung von Caprolactam anfallen und Caprolactam und Oligomere und Polymere des Caprolactams sowie Alkalimetallhydroxide enthalten, dadurch gekennzeichnet, daß man
(a) die Destillationsrückstände zur Schmelze bringt,
(b) Caprolactam fortlaufend aus der Schmelze mit überhitztem Wasserdampf und/oder Inertgas entfernt,
(c) nach dem Entfernen von Caprolactam die Schmelze abkühlt unter Erhalt eines Rückstandes,
(d) den so erhaltenen Rückstand in Wasser löst und
(e) die so erhaltene wäßrige Lösung der Verbrennung unter Bildung eines Alkalimetallcarbonats unterwirft.

2. Verfahren nach Anspruch 2, dadurch gekennzeichnet, daß man überhitzten Wasserdampf oder ein Inertgas mit einer Temperatur im Bereich von 250 bis 450°C verwendet.

3. Verfahren nach Anspruch 1 oder 2, dadurch gekennzeichnet, daß man als Alkalimetallhydroxid Natriumhydroxid und/oder Kaliumhydroxid verwendet.

## Claims

1. A process for recovering caprolactam and an alkali metal carbonate from distillation residues which are obtained in the purification of caprolactam and contain caprolactam and oligomers and polymers of caprolactam and also alkali metal hydroxides, which comprises
(a) melting the distillation residues,
(b) continuously removing caprolactam from the melt using superheated steam and/or inert gas,
(c) after removing caprolactam, cooling the melt and obtaining a residue,
(d) dissolving the residue thus obtained in water and
(e) subjecting the aqueous solution thus obtained to combustion with the formation of an alkali metal carbonate.

2. A process as claimed in claim 1, wherein superheated steam or an inert gas having a temperature in the range from 250 to 450°C is used.

3. A process as claimed in claim 1 or 2, wherein, as alkali metal hydroxide, sodium hydroxide and/or potassium hydroxide are used.

## Revendications

1. Procédé d'obtention de caprolactame et d'un carbonate de métal alcalin à partir de résidus de distillation, qui sont formés au cours de la purification de caprolactame et qui contiennent du caprolactame et des oligomères et des polymères du caprolactame ainsi que des hydroxydes de métal alcalin, caractérisé en ce que
(a) on amène à fusion les résidus de distillation,
(b) on élimine en continu du caprolactame à partir de la masse fondue à l'aide d'un gaz inerte et/ou de vapeur d'eau surchauffée,
(c) on refroidit la masse fondue après l'élimination du caprolactame, avec obtention d'un résidu,
(d) on dissout le résidu ainsi obtenu dans de l'eau, et
(e) on soumet la solution aqueuse ainsi obtenue à une combustion, avec formation d'un carbonate de métal alcalin.

2. Procédé suivant la revendication 1, caractérisé en ce qu'on utilise de la vapeur d'eau surchauffée ou un gaz inerte ayant une température de l'ordre de 250 à 450°C.

3. Procédé suivant l'une des revendications 1 et 2, caractérisé en ce que, comme hydroxyde de métal alcalin, on utilise de l'hydroxyde de sodium et/ou de l'hydroxyde de potassium.
